# EUROPÄISCHE PATENTSCHRIFT

(11) **EP 2 408 484 B1**
(45) Veröffentlichungstag und Bekanntmachung des Hinweises auf die Patenterteilung: **15.05.2013**
(21) Anmeldenummer: 10710296.4
(22) Anmeldetag: 19.03.2010
(51) Int. Cl.: A61L 2/18, A61L 2/24, A61C 1/00, A61C 19/00

(54) **GERÄT ZUM DESINFIZIEREN, STERILISIEREN UND/ODER PFLEGEN VON ÄRZTLICHEN, INSBESONDERE ZAHNÄRZTLICHEN INSTRUMENTEN**
DEVICE FOR DISINFECTING, STERILIZING AND/OR MAINTAINING MEDICAL, IN PARTICULAR DENTAL INSTRUMENTS
APPAREIL POUR DÉSINFECTER, STÉRILISER ET/OU ENTRETENIR DES INSTRUMENTS MÉDICAUX, EN PARTICULIER DES INSTRUMENTS DE DENTISTERIE

(30) Priorität: 20.03.2009 DE 102009014065; 17.02.2010 DE 102010002031
(43) Veröffentlichungstag der Anmeldung: 25.01.2012
(73) Patentinhaber: Kaltenbach & Voigt GmbH, 88400 Biberach (DE)
(72) Erfinder: HECKENBERGER, Hans, 88433 Aßmannshardt (DE); WIEK, Hans-Dieter, 88454 Hochdorf (DE); GUGEL, Bernd, 89070 Ulm (DE); STEMPFLE, Johann, 89284 Pfaffenhofen (DE); LOTT, Herbert, 88410 Bad Wurzach-Arnach (DE)
(74) Vertreter: Thun, Clemens
(86) Internationale Anmeldenummer: PCT/EP2010/053601
(87) Internationale Veröffentlichungsnummer: WO 2010/106159

(56) Entgegenhaltungen:
- EP-A1- 1 543 889
- EP-A2- 0 882 457
- EP-A2- 0 890 337
- EP-A2- 1 121 942
- WO-A2-2006/135647
- DE-A1- 4 021 790
- US-A1- 2004 190 883

## Beschreibung

Die vorliegende Erfindung betrifft ein Gerät, welches zum Desinfizieren, Sterilisieren und/oder Pflegen von ärztlichen Instrumenten vorgesehen ist. Insbesondere sollen mit dem Gerät zahnärztliche Instrumente aufbereitet werden.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führen die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Aus der Schrift WO 2006/135647 A2 ist ein Gerät zur Verteilung von Duft bekannt, bei dem ein Vorratsbehälter lediglich in einer bestimmten Orientierung auf ein Basiselement aufgesteckt werden kann. Aus der Schrift EP 1 543 889 A1 bist eine Reinigungs- und Desinfektionsmaschine bekannt, bei der Vorratsbehälter über Steckverbindungen angeschlossen werden können, durch die ein versehentliches Vertauschen der Behälter ausgeschlossen werden kann.

Aus der Schrift EP 1 121 942 A2 ist ein Gerät zum Desinfizieren von ärztlichen Instrumenten bekannt, das Anschlusseinrichtungen zum Anschließen eines Vorratsbehälters aufweist. Zur Identifizierung weist der Vorratsbehälter einen Strichcode auf. Die aus der Schrift EP 0 890 337 A2 bekannte Vorrichtung zum Reinigen und Sterilisieren von medizinischen Geräten weist eine Aufnahmekammer auf, die in ihrer Größe und Form an entsprechende Flüssigkeitsbehälter angepasst ist. Aus der Schrift DE 40 21 790 A1 ist ein Umfüllset bekannt, das eine Flaschenaufnahme für eine Portionsflasche aufweist, wobei für eine verwechslungsfreie Zuordnung die Flaschenaufnahme ein Schlosselement und die Portionsflasche ein Schlüsselelement aufweist. Ein Schloss- und Schlüsselsystem weist auch der aus der Schrift US 2004/0190883 A1 bekannte Duft-Verteiler auf. Aus der EP 0 882 457 A2 ist ein Lagersystem für Kassetten mit flüssigen Sterilisationsmitteln bekannt; zur Identifizierung der Kassetten wird ein Strichcode-Leser eingesetzt.

Der vorliegenden Erfindung liegt deshalb die Aufgabe zugrunde, ein neuartiges Gerät zum Desinfizieren, Sterilisieren und/oder Pflegen bzw. allgemein zum Aufbereiten von ärztlichen, insbesondere zahnärztlichen Instrumenten anzugeben, mit dem die oben genannten Nachteile vermieden werden.

Diese Aufgabe wird durch ein Gerät gemäß Anspruch 1 gelöst, welcher die Erfindung definiert.

Insbesondere betrifft die vorliegende Erfindung dabei die an dem Gerät vorgesehenen Anschlusseinrichtungen zum Anschließen von Vorratsbehältern für die von dem Gerät genutzten Medien. Es handelt sich hierbei um Reinigungs- und/oder Pflegemittel, also um diverse chemische Substanzen, die während den unterschiedlichen Aufbereitungsschritten eingesetzt werden. Eine Verwechslung der Substanzen, die in einer geänderten Reihenfolge der Zuführung resultiert, könnte zu schwerwiegenden Problemen, insbesondere zu Beschädigungen der aufzubereitenden Instrumente führen.

Die Anschlusseinrichtungen weisen ein mechanisches Kontrollelement auf, welches entsprechend dem anzuschließenden Vorratsbehälter unterschiedlich gestaltet und/oder positioniert ist. Bei diesem mechanischen Kontrollelement handelt es sich unter Anderem um einen Stift bzw. Vorsprung , der im montierten Zustand des Vorratsbehälters an dem Gerät in eine an dem Behälter ausgebildete Ausnehmung eingreift, wobei die Ausnehmung in Form einer taillierten Einschnürung gegeben ist. Alternativ wird als Kontrollelement auch eine Klammer eingesetzt werden, die in eine umlaufende Einschnürung in der Umfangswand des Vorratsbehälters eingreift.

Durch diese einfachen Maßnahmen erfolgt eine Kodierung der verschiedenen Behälter, sodass sichergestellt ist, dass die einzelnen Behälter jeweils nur an dem für sie vorgesehenen Anschluss montiert werden können. Fehler der oben beschriebenen Art können auf diese Weise vermieden werden, sodass die Betriebssicherheit des erfindungsgemäßen Geräts erhöht wird.

Nachfolgend soll die Erfindung anhand der beiliegenden Zeichnung näher erläutert werden. Es zeigen:
- Figur 1: in Schnittdarstellung eine Prozess- bzw. Spülkammer eines erfindungsgemäßen Geräts zum Desinfizieren, Sterilisieren und/oder Pflegen von zahnärztlichen Instrumenten;
- Figur 2: eine erste Variante zur mechanischen Kodierung eines Pflegemittelbehälters;
- Figur 3: eine zweite Möglichkeit zur Kodierung eines Pflegemittelbehälters;
- Figuren 4a und 4b: das Zusammenwirken eines Vorratsbehälters mit einem Kontrollelement in Form einer Klammer;
- Figuren 5a und 5b: eine dritte Variante zur mechanischen Kodierung eines Pflegemittelbehälters;
- Figuren 6a bis 6c: verschiedene denkbare Ausgestaltungen eines Pflegemittelbehälters; und
- Figuren 7 bis 12: weitere Varianten zum Identifizieren eines Pflegemittelbehälters.

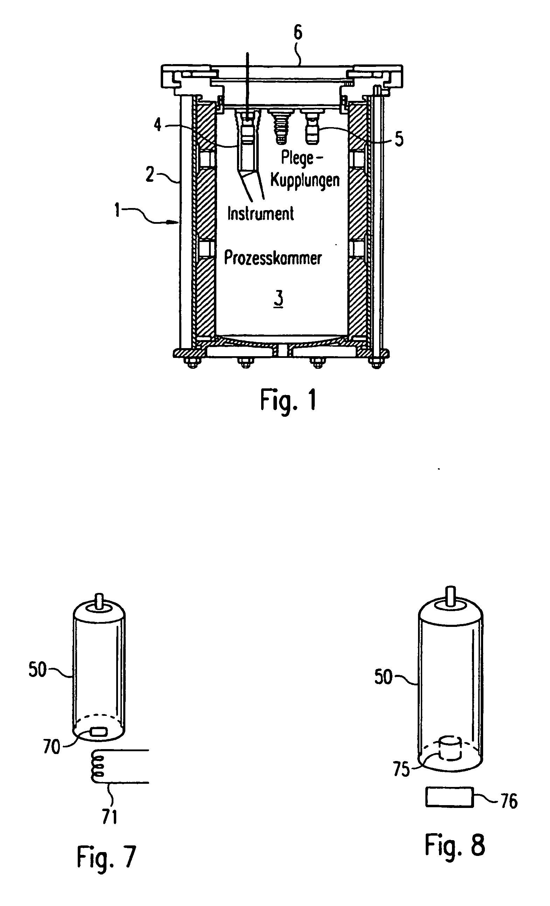

Figur 1 zeigt zunächst schematisch die Ausgestaltung eines Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist. Zentrales Element des erfindungsgemäßen Pflegegeräts 1 ist ein Druckbehälter 2, der eine Prozess- bzw. Spülkammer 3 umschließt. In dieser Spülkammer 3 sind während des Prozessablaufs die zu reinigenden bzw. pflegenden Instrumente 4 angeordnet. Die Anordnung der Instrumente 4 erfolgt hierbei mit Hilfe eines Instrumententrägers, auf dem mehrere Steckplätze bzw. Kupplungen 5 angeordnet sind. Vorzugsweise sind unterschiedliche Kupplungen 5 vorgesehen, sodass Instrumente 4 mit Kupplungssystemen verschiedener Hersteller aufbereitet werden können. Als Instrumententräger dient im vorliegenden Fall der Deckel 6 der Prozesskammer 3. Dieser Deckel 6 stellt die fluidische Ankopplung der zu reinigenden Instrumente 4 an ein Versorgungssystem sicher. Er wird durch eine Verriegelungsvorrichtung auf den Bund des Druckbehälters 2 geklemmt und gegenüber diesem abgedichtet. Über in den Deckel 6 integrierte Verbindungsrohre können dann die einzelnen Instrumente 4 und deren Kanäle einzeln oder gemeinsam mit einem Reinigungs- und/oder Pflegemittel beaufschlagt werden.

Nachfolgend soll zunächst allgemein der Prozessablauf bei der Reinigung und/oder Pflege der Instrumente 4 beschrieben werden. Hierbei wird vor dem Start der Aufbereitung die Druckdichtigkeit der Prozesskammer 3 überprüft. Dabei wird sichergestellt, dass der Deckel 6 richtig eingesetzt und mit dem Druckbehälter 2 verriegelt ist. Auch eine korrekte Verbindung der Fluidleitungen zwischen dem Deckel 6 und in dem Bund des Druckbehälters 2 verlaufenden Leitungen wird überprüft.

Zur Wasserversorgung des Geräts 1 wird Leitungswasser vorzugsweise mittels einer Osmose-Anlage mit oder ohne nachgeschalteten Mischbett-Ionenaustauscher filtriert, wobei die gelösten Salze entfernt werden. Das Wasser bei einer Qualität von <15 µS/cm wird in einen geräteseitigen Vorratsbehälter geleitet, wobei der Füllstand über einen Niveauschalter, der als Schwimmerschalter ausgestaltet ist, und die Güte über einen Leitwertsensor kontrolliert wird. Der Einlass in den Vorratsbehälter ist aus hygienischen Gründen mit einer sogenannten freien Fallstrecke ausgestaltet.

Beim Aufbereiten der Instrumente mit Hilfe des erfindungsgemäßen Geräts werden dann nacheinander die folgenden Schritte ausgeführt:

### a) Reinigen

Zunächst wird Wasser aus dem zuvor beschriebenen Vorratsbehälter in die Prozesskammer 3 geleitet, wobei dies über eine Pumpe oder durch Ansaugen über Vakuum erfolgen kann. In der Prozesskammer 3 wird das Wasser mit Hilfe von Heizelementen auf etwa 45°C aufgeheizt. Dabei wird darauf geachtet, dass die Temperatur nicht oberhalb von 45°C liegt, um ein Koagulieren von Eiweiß zu verhindern. Das Wasser wird ferner mit Hilfe einer Pumpe umgewälzt und über Sprühdüsen, welche an der Mantelfläche des Druckbehälters 2 oder in einem Zentraldom angebracht sind, auf die Außenflächen der Instrumente 4 gerichtet, um diese zu reinigen. Dabei kann das Reinigungswasser durch die Instrumente 4 und/oder die Spraykanäle der Instrumente 4 und/oder zur Außenreinigung durch die Sprühdüsen der Prozesskammer 3 geleitet werden.

Das Aufheizen des Waschmediums kann während der Umwälzung erfolgen, sodass die zu reinigenden Flächen zunächst mit kaltem Waschmedium gereinigt werden. Das Reinigungsmittel kann hierbei in Form von Pulver oder in Tablettenform in die Prozesskammer 3 zugegeben werden oder aus einem entsprechenden Vorratsbehälter zudosiert werden. Das Waschmedium kann dabei aus Tensiden bzw. Phosphaten bestehen und einen ph-Wert oberhalb von 10 aufweisen. Zur Beendigung des Waschvorgangs wird das Wasser aus dem Druckbehälter 2 abgelassen.

### b) Klarspülen - Neutralisation

In einem darauffolgenden Schritt wird dann das Wasser aus dem Vorratsbehälter in die Prozesskammer 3 geleitet und nun auf ca. 45°C bis 60°C aufgeheizt. Während des Umwälzenz des Wassers wird aus einem weiteren Vorratsbehälter Klarspüler bzw. Neutralisator zudosiert. Alternativ kann aufgrund der höheren Temperatur im Vergleich zu dem Schritt a) nunmehr auch eine zweite Komponente einer Reinigungstablette aufgelöst werden. Die Flüssigkeit wird wiederum parallel oder zeitversetzt bzw. im Intervallbetrieb durch die Instrumente 4 und die Spraykanäle geleitet bzw. über die Sprühdüsen auf die Außenflächen der Instrumente 4 gerichtet. Als Klarspüler bzw. Neutralisator kommt insbesondere Phosphorsäureester mit einem ph-Wert von 3 bis 5 zur Anwendung.

Die Flüssigkeit kann wiederum aus dem Druckbehälter in die Kanalisation abgelassen werden oder verbleibt im Behälter, um beim späteren Pflegevorgang aus den Instrumenten 4 austretendes überschüssiges Pflegemittel aufzunehmen bzw. um die ölige Instrumentenaußenfläche mit warmer Flüssigkeit kurz abzuspülen. In diesem Fall wird die Flüssigkeit erst nach dem Pflegevorgang abgelassen, wobei es hilfreich sein kann, die Instrumente 4 mit Druckluft zu beaufschlagen, um ein Eindringen von Sprühwasser in das Innere der Instrumente 4 zu verhindern.

### c) Pflegen

In einem dritten Schritt wird aus einem Pflegemittelvorratsbehälter Pflegemittel in das Instrumenteninnere geleitet, sodass die Getriebe und Lagerstellen geschmiert werden. Das Pflegemittel kann dabei in flüssiger Form als Öl oder aus einer Druckdose in einen Druckluftstrahl injektiert werden. Es ist auch möglich, das Öl über das in der Druckdose enthaltene Treibmittel aufzuschäumen und das Instrumenteninnere mit diesem Öl-Luft-Schaum zu füllen. Die Luftbläschen kollabieren in diesem Fall verhältnismäßig rasch, sodass das Öl im gesamten Instrumenteninneren einen gleichförmigen dünnen Ölfilm bildet. Als Schmierstoffe kommen biologisch abbaubare Fettsäure-Esteröl/Weißöl-Gemische zur Anwendung.

### d) Abspülen

Nachdem zuvor beschriebenen Pflegevorgang können die Instrumente an der Außenfläche mit der noch im Behälter befindlichen Klarspülflüssigkeit abgespült werden. Alternativ hierzu wird über eine Pumpe frisches Wasser aus dem Vorratsbehälter der Prozesskammer 3 zugeführt und über die Sprühdüsen auf die Instrumentenaußenflächen gerichtet.

### e) Sterilisation - Vorvakuum

Zum Sterilisieren der Instrumente wird der Prozesskammer 3 frisches Wasser aus dem Vorratsbehälter zugeführt. In der Prozesskammer 3 ist zur Entlüftung eine Vakuumeinrichtung angeschlossen, wobei der Druck innerhalb der Prozesskammer 3 überwacht bzw. registriert wird.

Mit Hilfe der Vakuumeinrichtung wird die Luft aus der Prozesskammer 3 abgesaugt. Das Vakuum wird durch Aufheizen des Wassers über Heizelemente bis auf Atmosphärendruck abgebaut. Die Prozesskammer 3 wird dann mit Wasserdampf befüllt, wobei sich dieser Vorgang je nach Sterilisationsprogramm mehrmals wiederholen kann.

Das verdampfte Wasservolumen kann bei jedem Vakuumzyklus nachgefüllt werden, wobei alternativ hierzu auch die gesamte für die Dampferzeugung erforderliche Wassermenge gleich zu Beginn des Sterilisationszyklus in die Prozesskammer 3 eingebracht werden kann.

Alternativ zur Dampferzeugung über in der Prozesskammer 3 befindliche Heizelemente kann Wasserdampf zum Druckausgleich bei der Entlüftung bzw. zur Sterilisation auch aus einem außerhalb der Prozesskammer 3 befindlichen Dampfdruckkessel zugeführt werden.

### f) Trocknung und Kühlung

Nach Abschluss der Sterilisation werden die Instrumente 4 getrocknet, in dem der in der Prozesskammer 3 befindliche Wasserdampf zur Kondensation gebracht wird. Dies wird dadurch erzielt, dass die Behälterwand oder in dem Behälter befindliche Elemente gekühlt werden, beispielsweise indem aus dem Vorratshälter entnommenes Wasser durch sie geleitet wird. Dabei kann das Wasser kontinuierlich oder intervallförmig zugeführt werden. Nach Beendigung des Kühlvorgangs wird das Wasser abgeleitet. Da nunmehr innerhalb der Kamer 3 eine Temperatur unterhalb von 50°C vorliegt, kann der Deckel 6 geöffnet werden. Der Aufbereitungszyklus für die Instrumente 4 ist hierdurch abgeschlossen.

Aus der obigen Schilderung ergibt sich, dass mit dem Gerät 1 eine vollautomatische Aufbereitung zahnärztlicher Instrumente ermöglicht wird. Eingriffe durch Bedienpersonal sind nicht erforderlich, sodass ein sehr komfortables System vorliegt. Dabei kann selbstverständlich auch von dem geschilderten Auflauf zum Aufbereiten der Instrumente abgewichen werden.

Dem oben geschilderten Verfahrensablauf ist ferner entnehmbar, dass bei der Aufbereitung der Instrumente verschiedene chemische Substanzen zur Reinigung, Pflege und Desinfektion zum Einsatz kommen. Die Substanzen werden in unterschiedlichen Zustandsformen (fest, pulvrig oder flüssig) und Verpackungen (Spraydosen, Flüssigdispenser-Flaschen oder Tabletten) angeboten und eingesetzt. Eine Kennzeichnung der unterschiedlichen Produkte erfolgt dabei üblicherweise durch entsprechende Aufdrucke auf der Verpackung bzw. dem Gefäß.

Das das Gerät bedienende Personal muss bei der Anwendung die verschiedenen Kennzeichnungen der Verpackungen beachten, beispielsweise die richtige Kartusche bzw. Spraydose in die zugehörige Anschlusseinrichtung einsetzen. Eine Verwechslung der Substanzen hätte eine geänderte Reihenfolge der Anwendung zur Folge, was im schlimmsten Fall zu einer Beschädigung der aufzubereitenden Instrumente, zumindest jedoch zu einer Beeinträchtigung der angestrebten Wirkung führen kann. Hieraus kann sich ein erheblicher Schaden sowohl für den Patienten als auch den Anwender ergeben.

Um diese Nachteile zu vermeiden, wird erfindungsgemäß vorgeschlagen, das Gerät derart auszugestalten, dass es selbständig die verschiedenen Vorratsbehälter erkennt bzw. eine fehlerhafte Anordnung verhindert.

Dabei ist bei einer ersten Variante vorgesehen, die Gefäße bzw. Vorratsbehälter für die unterschiedlichen Substanzen mit einem mechanischen Kodierungssystem zu versehen, welches auf unterschiedlichen äußeren Formen der Gefäße beruht. Das Gerät ist dann mit einem entsprechenden Erkennungssystem bzw. Blockiersystem ausgestattet, welches letztendlich dazu führt, dass die Vorratsbehälter lediglich an der hierfür vorgesehenen Anschlusseinrichtung des Geräts angeordnet werden können. Hierzu können die Vorratsbehälter unterschiedliche Außenformen und/oder unterschiedliche Größen aufweisen. Auch die Ausstattung mit verschiedenen Anschlussgewinden oder verschiedenen Durchmessern für die Anschlussstutzen wäre denkbar.

Der Vorratsbehälter in Form einer Kartusche oder Dose wird üblicherweise durch Einschrauben in ein Anschlussventil, Einsetzen in ein Anschlussventil und Halten über Magnetkräfte, Einpressen in das Einschlussventil über einen Hubmechanismus oder Andrücken in das Anschlussventil bzw. Halten über Federkräfte an die entsprechende Anschlusseinrichtung angeschlossen. Es wird nunmehr vorgeschlagen, an dem Vorratsbehälter je nach Substanz auf unterschiedlicher Höhe eine taillienförmige Einschnürung anzubringen, in welche beim Einsetzen in die Anschlusseinrichtung ein geräteseitiges Kodieridentifizierungselement bzw. Kontrollelement in Form eines Bügels, einer Klammer, einer Rachenlehre oder eines Stifts eingreift. Ist die geforderte Einschnürung an dem Vorratsbehälter nicht vorhanden bzw. an einer falschen Stelle angeordnet, so hat dies zur Folge, dass der Behälter nicht an die Anschlusseinrichtung angeschlossen werden bzw. eine an dem Gerät vorgesehene Tür nicht geschlossen werden kann, was letztendlich zur Folge hat, dass das Gerät nicht in Betrieb genommen werden kann. Die Zuführung falscher Medien ist auf diesem Wege ausgeschlossen.

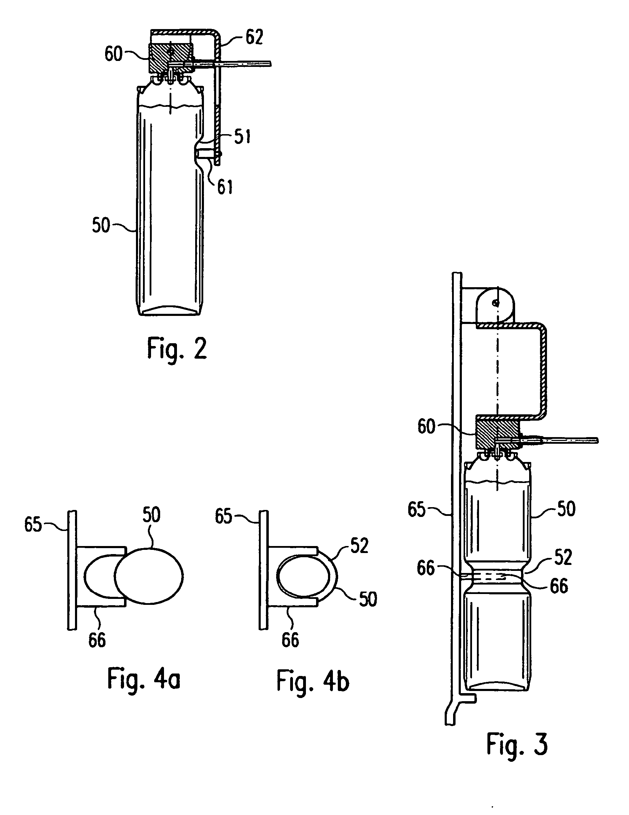

Eine erste Ausführungsform dieses erfindungsgemäßen Gedankens ist in Figur 2 dargestellt. Eine mit dem Bezugszeichen 50 versehene Dose wird hierbei in den Anschluss 60 des Geräts eingesetzt, wobei der Anschluss 60 ein Kontroll- bzw. Identifizierungselement 61 in Form eines Stifts aufweist, der an einem Bügel 62 befestigt ist. Die Dose 50 weist an ihrem Außenumfang eine Einschnürung 51 auf, welche in Höhe des Stifts 61 ausgebildet ist, so dass dieser in die Einschnürung 51 eingreifen kann. Die Breite der Einschnürung 50 ist dabei derart bemessen, dass die Dose 50 in Achsrichtung ausreichend Spiel zum vollständigen Einschrauben bzw. Einsetzen hat.

Ist hingegen die Einschnürung 51 nicht in Höhe des Stifts 61 angeordnet bzw. gar nicht vorhanden, so handelt es sich um einen falschen Vorratsbehälter, der nicht zum Einsetzen in den dargestellten Anschluss 60 vorgesehen ist. Da der Stift 61 nun ein Einsetzen des falschen Behälters verhindert, wird dementsprechend sichergestellt, dass lediglich Behälter mit dem richtigen Inhalt angeschlossen werden können.

Eine zweite Variante ist in den Figuren 3 und 4a bzw. 4b dargestellt. Hierbei wird eine Dose 50 in einen nicht schwenkbaren Anschluss 60 eingeschraubt, sodass sie sich bereits in der Einsatzposition befindet. Das Einsetzen einer falschen Dose wird nunmehr dadurch angezeigt, dass an einer Gehäuseklappe 65 eine rachenförmige Klammer 66 angeordnet ist. Diese ist wiederum dazu vorgesehen, in eine entsprechende umlaufende Einschnürung 52 an dem Mantel der Dose 50 einzugreifen. Ist die Einschnürung 52 in falscher Höhe ausgebildet, ergibt sich die Konfiguration in Figur 4a, bei der die rachenförmige Klammer 66 ein Schließen der Gehäuseklappe 65 verhindert. Für den Fall, dass die korrekte Dose 50 eingesetzt wurde, ergibt sich hingegen die Konfiguration gemäß Figur 4b, bei der die Gehäuseklappe 65 vollständig geschlossen werden kann. Als zusätzliche Sicherheitsmaßnahme kann dabei über elektrische Schalter oder Sensoren das vollständige Schließen der Gehäuseklappe 65 überwacht werden. Ist diese Klappe 65 nicht vollständig geschlossen, so wird der Betrieb des Geräts verhindert.

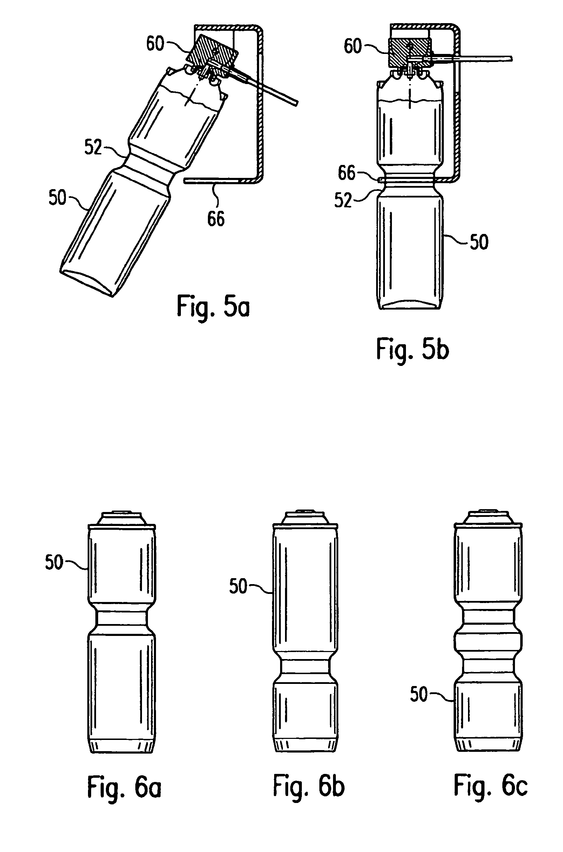

Eine dritte Ausführungsform ist in den Figuren 5a und 5b dargestellt, bei der eine Dose 50 in einen ausschwenkbaren Anschluss 60 eingeschraubt werden kann und dann in die Einsatzposition in Figur 5b zurückgeschwenkt wird. Wiederum kommt als Kontrollelement eine rachenförmige Klammer 66 zum Einsatz, welche bei Verwendung des korrekten Vorratsbehälters in die umlaufende Einschnürung 52 eingreift. Bei dem Versuch, eine falsche Dose anzuordnen wird hingegen ein Zurückschwenken verhindert.

Alle drei beschriebenen Varianten beruhen also darauf, am Anschluss 60 des Geräts ein Kontrollelement anzubringen, welches mit einer entsprechenden Formgebung des Vorratsbehälters zusammenwirkt, um ein irrtümliches Einsetzen eines falschen Vorratsbehälters zu verhindern. Dabei können unterschiedlichste Einschnürungen bzw. Ausnehmungen in der Umfangswand des Behälters 50 vorgesehen werden, wie die Figuren 6a bis 6c zeigen.

Die Figuren 7 bis 12 zeigen alternative Lösungen, die Identität des angeschlossenen Vorratsbehälters zu überprüfen. Diese Lösungen sollen nachfolgend erläutert werden.

Bei der Variante gemäß Figur 7 ist der Vorratsbehälter 50 mit einem Transponder 70 versehen. Die Anschlusseinrichtungen des Geräts wiederum weisen ein schematisch dargestelltes Lesegerät 71 auf, um mit dem Transponder 70 zu kommunizieren. Der Transponder 70 befindet sich vorzugsweise außen am Boden der Dose 50, er kann sich jedoch auch an der Mantelfläche befinden. Die Trägerfrequenz für die Kommunikation liegt etwa im Bereich von 125 kHz. Bei der Benutzung mehrerer Dosen erhält jede Dose mit Hilfe des Transponders eine eigene Kodierung, um eine Fehlbestückung in dem Gerät zu unterbinden. Der entnommene Inhalt bzw. die Zyklenzahl der Entnahme wird auf den Transponder zurückgeschrieben, so dass auch auf den noch verfügbaren Inhalt der Dose zurückgeschlossen werden kann. Dieser könnte beispielsweise auch auf dem Gerät angezeigt werden, um rechtzeitig die Beschaffung eines Ersatzvorratsbehälters zu initiieren. Dies stellt einen zusätzlichen Vorteil neben der Vermeidung einer Fehlbestückung dar.

Bei der Variante gemäß Figur 8 ist der Vorratsbehälter 50 mit einem Magneten 75 versehen, der den Inhalt der Dose charakterisiert. Die Anschlusseinrichtungen des Geräts weisen einen entsprechenden Sensor 76, beispielsweise einen Hallsensor auf, über den das Magnetfeld des Magneten 75 erfasst werden kann. Durch die Wahl geeigneter Magneten kann auch hier der Inhalt des Vorratsbehälters 50 charakterisiert werden. Ferner könnte der Magnet 75 gleichzeitig dazu benutzt werden, ein in dem Medienweg der Anschlusseinrichtungen befindliches Ventil zu betätigen bzw. freizugeben. Dadurch wird zusätzlich die Betriebssicherheit erhöht.

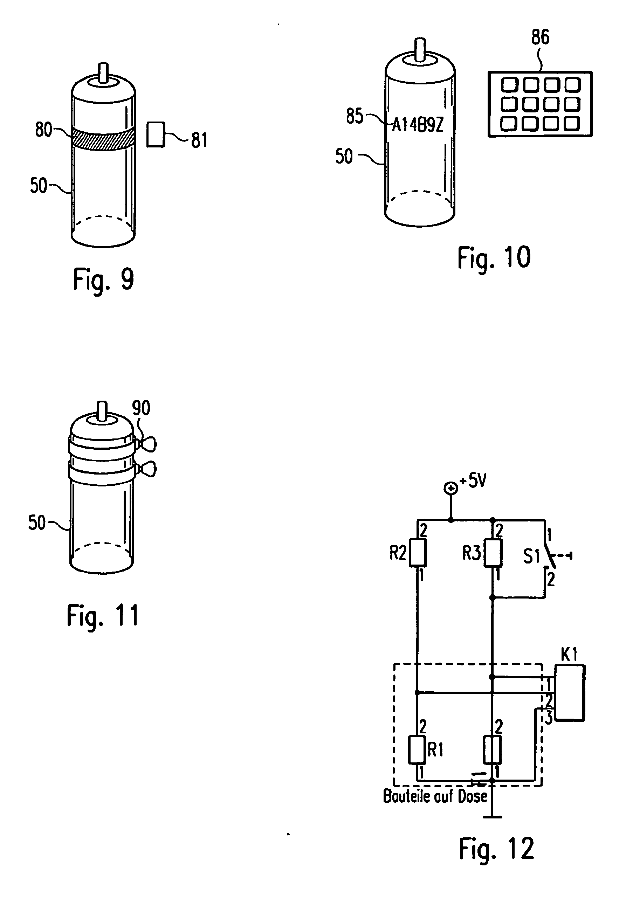

Bei der Variante gemäß Figur 9 ist an der Außenseite des Vorratsbehälters eine optische Markierung aufgebracht, die im dargestellten Ausführungsbeispiel durch einen Farbring 80 gebildet ist. Über einem am Gerät 1 befindlichen Farbsensor 81 kann dann die aufgedruckte Farbe an dem Vorratsbehälter 50 erkannt werden. Durch den Einsatz verschiedener Farben ist es wiederum möglich, verschiedene Vorratsbehälter zu unterscheiden. Alternativ hierzu wäre es auch denkbar, ein Bild bzw. allgemein ein optisches Element an der Außenseite des Vorratsbehälters anzuordnen und dieses durch entsprechende Mittel des Geräts zu erfassen und zu bewerten. Mit Hilfe einer Digitalkamera sowie einer entsprechenden Mustererkennungssoftware könnte dann das an der Dose befindliche Symbol ermittelt werden, um gegebenenfalls eine Fehlbestückung zu erkennen bzw. den Einsatz des richtigen Vorratsbehälters zu erkennen.

Bei der Variante gemäß Figur 10 ist an der Mantelfläche des Vorratsbehälters ein Code 85 aufgedruckt. Dieser muss nach dem Anbringen des Behälters von dem Benutzer über ein an dem Gerät befindliches Eingabefeld 86 eingegeben werden. Eine entsprechende Kontrolleinrichtung an dem Gerät überprüft dann die Richtigkeit des Codes, wobei im Falle eines positiven Ergebnisses der Behälter freigeschaltet wird. Intern kann dieser eingegebene Code dann auch gespeichert werden, so dass er für weitere Freischaltungen nicht mehr zur Verfügung steht. Auf diesem Wege wird auch eine mehrmalige Benutzung des Behälters verhindert.

Bei der Variante gemäß Figur 11 enthält der Vorratsbehälter 50 eine Art Schlüssel, der mit einem entsprechenden Schloss bzw. Schlüsselzylinder in dem Gerät zusammenwirkt. Der Schlüssel 90 des Vorratsbehälters 50 kann also genutzt werden um den Weg zur Dose 50 zu öffnen oder um die Dose aufzunehmen. Dies kann mechanisch, elektronisch oder elektrisch erfolgen. Dabei könnte der Schlüssel als Felgenschluss oder Ähnliches realisiert werden, oder so ausgestaltet sein, dass er gleichzeitig als Dosenaufnahme für das Gerät dient.

Bei einer in den Figuren nicht dargestellten Variante ist ferner vorgesehen, dass der Inhalt des Vorratsbehälters über seine physikalischen und/oder chemischen Eigenschaften identifiziert wird. Die Anschlusseinrichtung weist hierzu einen Sensor auf, über den beispielsweise der Leitwert, der Berechnungsindex, die Farbe, die Fluoreszenz und dergleichen bei einer erstmaligen Entnahme des Mediums bestimmt wird. Auf diesem Weg kann wiederum eine Fehlbestückung erkannt werden.

Schließlich zeigt Figur 12 eine Variante, bei der der Inhalt des Vorratsbehälters über einen elektrischen Schaltkreis identifiziert wird. Dieser enthält zumindest ein passives Bauelement, beispielsweise einen Widerstand, eine Spule oder einen Kondensator. Der Schaltkreis ist auf einer Platine angeordnet, die dem Behälter zugeordnet ist. Er weist eine Kontaktverbindung auf, welche einen Anschluss an die Anschlusseinrichtungen des Aufbereitungsgeräts ermöglicht.

Bei der in Figur 12 dargestellten Variante wird beispielsweise beim Einsetzen des Vorratsbehälters über den Verbinder K1 eine Verbindung zu dem Gerät hergestellt. Die Widerstände R1 und R2 ergeben einen Spannungsteiler, wobei dann bei Anlegen einer vorgegebenen Spannung am Verbinder K1 über den PIN2 die Spannung ermittelt werden kann. Durch Nutzung verschiedener Werte für die Widerstände können dann die Vorratsbehälter entsprechend kodiert werden.

Der Schaltkreis weist ferner ein Sicherungselement auf, dessen Zustand einmalig veränderbar ist. Dieses Element kann beispielsweise durch eine Diode im Überlastbereich gebildet werden.

Über den Eingang 1 am Steckverbinder K 1 kann ermittelt werden, ob die Sicherung noch intakt ist. Wird der Vorratsbehälter vollständig entleert, so kann über den Schalter S 1 die Sicherung ausgelöst werden. Die durchgebrannte Sicherung dient dann zur Kennzeichnung dafür, dass der Behälter leer ist. Falls versucht wird, die Platine auf eine andere Dose zu übertragen, kann dies vom Gerät anhand der durchgebrannten Sicherung erkennt werden. Anstelle der Diode könnte auch ein dünnes Leiterbahnsegment, das durchbrennt, oder ein Widerstand mit sehr geringer Belastbarkeit genutzt werden.

Alle beschriebenen Varianten erlauben es, den Behälter mit dem darin befindlichen Medium eindeutig zu charakterisieren, so dass eine Fehlbestückung des Geräts zuverlässig vermieden wird.

## Patentansprüche

1. Gerät (1) zum Desinfizieren, Sterilisieren und/oder Pflegen von ärztlichen, insbesondere zahnärztlichen Instrumenten (4), aufweisend Anschlusseinrichtungen (60) zum Anschließen von Vorratsbehältern (50) für von dem Gerät (1) genutzte Substanzen, insbesondere Reinigungs- oder Pflegemittel, sowie Vorratsbehälter (50), wobei die Anschlusseinrichtungen (60) Kontroll- bzw. Identifizierungsmittel aufweisen, welche eine Identifizierung des Vorratsbehälters (50) ermöglichen, und wobei die Kontroll- bzw. Identifizierungsmittel ein mechanisches Kontroll- bzw. Identifizierungselement (61, 66) aufweisen, welches entsprechend dem anzuschließenden Vorratsbehälter (50) unterschiedlich gestaltet und/oder positioniert ist,
**dadurch gekennzeichnet,**
**dass** am Vorratsbehälter je nach Substanz auf unterschiedlicher Höhe eine taillienförmige Einschnürung angebracht ist, in welche beim Einsetzen in die Anschlusseinrichtung das geräteseitige Kontroll- bzw. Identifizierungselement (61, 66) (61, 66) in Form eines Vorsprungs, Bügels, einer Klammer, einer Rachenlehre oder eines Stifts eingreift,
oder dass es sich bei dem mechanischen Kontroll- bzw. Identifizierungselement (61, 66) um eine Klammer handelt, welche im montierten Zustand des Vorratsbehälters (50) in eine umlaufende Einschnürung in der Umfangswand des Vorratsbehälters (50) eingreift.

2. Gerät nach Anspruch 1,
**dadurch gekennzeichnet,**
**dass** das mechanische Kontroll- bzw. Identifizierungselement (61, 66) an einer Klappe bzw. Tür (65) des Geräts (1) angeordnet ist und das Gerät (1) ferner eine Einrichtung zum Kontrollieren des vollständigen Schließens der Klappe bzw. Tür (65) aufweist.

## Claims

1. A device (1) for disinfecting, sterilizing and/or maintaining medical, more particularly dental, instruments (4), having connection units (60) for connecting storage containers (50) for substances, more particularly cleaning or maintenance products, utilized by the device (1), and storage containers (50), wherein the connection units (60) have checking or identification means (61, 66) that allow identification of the storage container (50),
and wherein the checking or identification means have a mechanical checking or identification element (61, 66), which has a different design and/or positioning in accordance with the storage container (50) to be connected,
**characterized in that**
a waisted constriction is applied to the storage container at different heights, depending on the substance, with the device-side checking or identification element (61, 66) in the form of a projection, bracket, a clamp, a gap gauge or a pin engaging into the constriction upon insertion into the connection unit, or
the mechanical checking or identification element (61, 66) is a clamp, which engages into a circumferential constriction in the peripheral wall of the storage container (50) in the assembled state of the latter.

2. A device according to claim 1,
**characterized in that**
the mechanical checking or identification element (61, 66) is arranged on a flap or door (65) of the device (1), and the device (1) furthermore has a unit for checking the complete closure of the flap or door (65).

## Revendications

1. Appareil (1) servant à désinfecter, stériliser et/ou à entretenir des instruments (4) médicaux, en particulier des instruments de dentisterie, présentant des dispositifs de raccordement (60) servant à raccorder des réservoirs (50) destinés à des substances utilisés par l'appareil (1), en particulier des produits de nettoyage ou d'entretien et des réservoirs (50), sachant que les dispositifs de raccordement (60) présentent des moyens de contrôle ou d'identification (61, 66), lesquels permettent d'identifier le réservoir (50), sachant que les moyens de contrôle au d'identification présentent un élément de contrôle ou d'identification (61, 66) mécanique, lequel est configuré et/ou positionné différemment selon le réservoir (50) à raccorder,
**caractérisé en ce**
**qu'**est pratiqué un rétrécissement en forme de taille sur le réservoir selon la substance à une hauteur différente, avec lequel vient en prise lors de l'introduction dans le dispositif de raccordement, l'élément de contrôle ou d'identification (61, 66) côté appareil sous la forme d'une saillie, d'un étrier, d'un serre-joint, d'un calibre à fourches ou d'une tige, ou en ce que
l'élément de contrôle ou d'identification (61, 66) mécanique est un serre-joint, qui vient en prise lorsque le réservoir (50) se trouve dans l'état monté avec un rétrécissement périphérique dans la paroi périphérique du réservoir (50).

2. Appareil selon la revendication 1,
**caractérisé en ce**
**que** l'élément de contrôle ou d'identification (61, 66) mécanique est disposé sur une trappe ou une porte (65) de l'appareil (1), et en ce que l'appareil (1) présente en outre un dispositif servant à contrôler la fermeture complète de la trappe ou de la porte (65).
